# EUROPEAN PATENT APPLICATION

(11) **EP 4 785 916 A1**
(43) Date of publication of application: **05.08.2026**
(21) Application number: 24820251.7
(22) Date of filing: 10.06.2024
(51) Int. Cl.: A61F 13/514, A61F 13/49, A61F 13/51, A61F 13/53, A61F 13/56, A61F 13/475

(54) **DISPOSABLE DIAPER**

(30) Priority: 25.09.2023 JP 2023161590; 09.03.2024 JP 2024036422
(71) Applicant: Koyo Corporation, Yokohama-shi, Kanagawa 236-0004 (JP)
(72) Inventor: SHIRAI, Atsuko, Yokohama-shi Kanagawa 2360004 (JP); MATSUMIYA, Munetada, Yokohama-shi Kanagawa 2360004 (JP); OGURA, Nami, Yokohama-shi Kanagawa 2360004 (JP)
(74) Representative: Samson & Partner Patentanwälte mbB
(86) International application number: PCT/JP2024/021069
(87) International publication number: WO 2025/069583

(57) **Abstract**

The disposable diaper 1 includes the left and right guidelines 17 and 18, which have left and right straight portions 17b and 18b extending in the longitudinal direction at positions overlapping the left and right inner elastic members 12L and 12R in the middle section M as portions that are applied along the wearer's thigh bases, and which have left and right back oblique portions 17c and 18c extending obliquely from positions overlapping the left and right outer elastic members 11L and 11R in the back transition section FM to the back ends of the left and right straight portions 17b and 18b as portions that are applied along the wearer's gluteal grooves, and the back transition section FM and the back section B of the body 2 have a portion in which the outer elastic members 11L and 11R, the inner elastic members 12L and 12R and the back end elastic members 13 are arranged in an approximately arc shape ,which fits along the wearer's buttocks when worn and which provides the bulge structure 16 with an excrement storage space between an inner surface of the portion and the buttocks.

## Description

### TECHNICAL FIELD

The present invention relates to disposable diapers, and more particularly, to tape-type disposable diapers.

### BACKGROUND ART

A tape-type disposable diaper disclosed in Patent Literature 1 includes a target tape provided on an exterior surface of a ventral member, fastening tapes protruding from left and right edges of a dorsal member and attachable to the target tape, left and right elastic members provided along left and right edges of the diaper from a part of the ventral member through a middle member to a front half of the dorsal member, and an end flap elastic member provided in a left and right direction in a back end center of the dorsal member in an area without absorber. However, since the elastic member of the diaper is arranged as described above, it is difficult to wear the diaper in a proper position so as to fit a rounded shape of wearer's buttocks and prevent leakage of excrement.

Tape-type disposable diapers are often used by caregivers to put on elderly people and patients, who are lying down. As disclosed in Patent Literature 1, tape-type disposable diapers usually have an overall shape of a rectangle with a narrowed middle section in the longitudinal direction. The diaper of the above shape is applied to the buttocks, crotch, and lower abdomen of the human body, folded, aligned, and secured with the tape provided on the diaper to complete its attachment to the human body. However, since the shape of the human body is three-dimensional and complex, it is not easy to properly wear a tape-type disposable diaper with the above-mentioned simple, flat shape without causing leakage of excrement. In particular, it is difficult to apply the diaper along wearer's groin, thigh bases, and gluteal grooves, and it is also difficult to determine an appropriate tape fastening position to maintain such a state.

### RELATED ART DOCUMENT

### PATENT LITERATURE

Patent Literature 1: Japanese Patent No. 5603391

### SUMMARY OF INVENTION

### TECHNICAL PROBLEM

The present invention has an object to provide a disposable diaper that is easy to fit properly to prevent leakage of excrement.

### SOLUTION TO PROBLEM

According to one aspect of the present invention, a disposable diaper includes a body that envelops an absorber with a plurality of sheets, the body having an overall shape in which a width of a middle section of a longitudinal direction of a rectangle is narrowed, the body having a front section that is one end in the longitudinal direction, a back section that is another end and is longer than the front section, a front transition section that gradually narrows from the front section to the middle section, and a back transition section that gradually narrows from the back section to the middle section; tapes protruding outward from left and right edges of the back section of the body; fastening units provided at protruding ends of the tapes and attachable to an exterior surface of the front section or the front transition section of the body; outer elastic members comprising of a group of left and right thread-like elastic materials, the outer elastic members being arranged in the longitudinal direction along the left and right edges in the middle section of the body, being arranged obliquely along the left and right edges in the back transition section, being arranged in the longitudinal direction along the left and right edges in the back section excluding a back end section, and being arranged obliquely toward a left-right centerline of the body in the back end section; inner elastic members comprising of a group of left and right thread-like elastic materials provided closer to the left-right centerline than the left and right outer elastic members, the inner elastic members being arranged in the longitudinal direction at a distance from the outer elastic members in the middle section of the body, being arranged obliquely toward the back section so as to gradually approach the outer elastic members in the back transition section, being arranged in the longitudinal direction along the outer elastic members in the back section excluding the back end section, and being arranged obliquely toward the left-right centerline along the outer elastic members in the back end section; back end elastic members that are arranged in a width direction so as to couple between the left and right inner elastic members adjacent to the back edge of the back section of the body; and left and right guidelines that are visible on the exterior surface of the front transition section, the middle section and the back transition section of the body and are applied along wearer's groins, thigh bases and gluteal grooves. The left and right guidelines have left and right straight portions extending in the longitudinal direction at positions overlapping the left and right inner elastic members in the middle section as portions that are applied along the wearer's thigh bases. The left and right guidelines have left and right back oblique portions extending obliquely from positions overlapping the left and right outer elastic members in the back transition section to the back ends of the left and right straight portions as portions that are applied along the wearer's gluteal grooves. The back transition section and the back section of the body have a portion in which the outer elastic members, the inner elastic members and the back end elastic members are arranged in an approximately arc shape, which fits along the wearer's buttocks when worn and which provides a bulge structure with an excrement storage space between an inner surface of the portion and the buttocks.

In the first aspect of the present invention, the left and right guidelines may have left and right front oblique portions extending obliquely from positions overlapping the left and right outer elastic members in the front transition section to the front edges of the left and right straight portions as portions that are applied along the wearer's groins.

In the first aspect of the present invention, a center point indicating a center position of each of the portions that are applied along the wearer's thigh bases in the left and right guidelines may be visible on the exterior surface of the body.

In the first aspect of the present invention, a centerline indicator located on the left-right centerline of the body may be visible on the exterior surface of the body.

In the first aspect of the present invention, left and right reference marks located symmetrically with respect to the left-right centerline as landmarks for attachment positions of the fastening units may be visible on the exterior surface of the body.

In the first aspect of the present invention, a group of graphic marks formed symmetrically or regularly arranged symmetrically with respect to the left-right centerline and an orthogonal line to the left-right centerline in the front section of the body may be visible on the exterior surface of the body.

In the first aspect of the present invention, a pattern expanding from the middle section to the back section of the body may be visible only on the exterior surface within the area surrounded by the left and right inner elastic members in the back section.

In the first aspect of the present invention, the disposable diaper may further include a target tape where the fastening unit is attachable and is provided on the exterior surface in the front section or the front transition section of the body.

In the first aspect of the present invention, the disposable diaper may further include a flap whose base end is secured adjacent to the back edge of the back section of the body and whose free end has a flap elastic member arranged in the left-right direction, and the back end elastic member is provided within the base end of the flap.

In the first aspect of the present invention, the disposable diaper may further include left and right standing sheets for standing on each line along the left and right edges of the middle sections of the body and left and right standing elastic members arranged along each free edge of the left and right standing sheets.

In the first aspect of the present invention, the absorber may have two recesses and a bulging portion between the recesses on each of the left and right edges.

In the first aspect of the present invention, the absorber may have two recesses and a bulging portion between the recesses on each of the left and right edges, and the left and right inner elastic members may be arranged adjacent to the base of the left and right bulging portions.

The present invention is not limited to the above-mentioned objects or aspects. A more complete understanding of the present invention and many of the advantages thereof will be readily apparent from the following detailed description and the associated drawings.

### BRIEF DESCRIPTION OF DRAWINGS

FIG. 1 is a bottom view of a disposable diaper according to a first embodiment of the present invention with elastic member not exerting elasticity;
FIG. 2 is a schematic end view of the disposable diaper taken along the line II-II of FIG. 1;
FIG. 3 is a schematic end view of the disposable diaper taken along the line III-III of FIG. 1;
FIG. 4 is a photograph showing a back of a disposable diaper according to an embodiment of the present invention when worn;
FIG. 5 is a photograph showing a front of a disposable diaper according to an embodiment of the present invention when worn;
FIG. 6 is a bottom view of a disposable diaper according to a second embodiment of the present invention with elastic member not exerting elasticity;

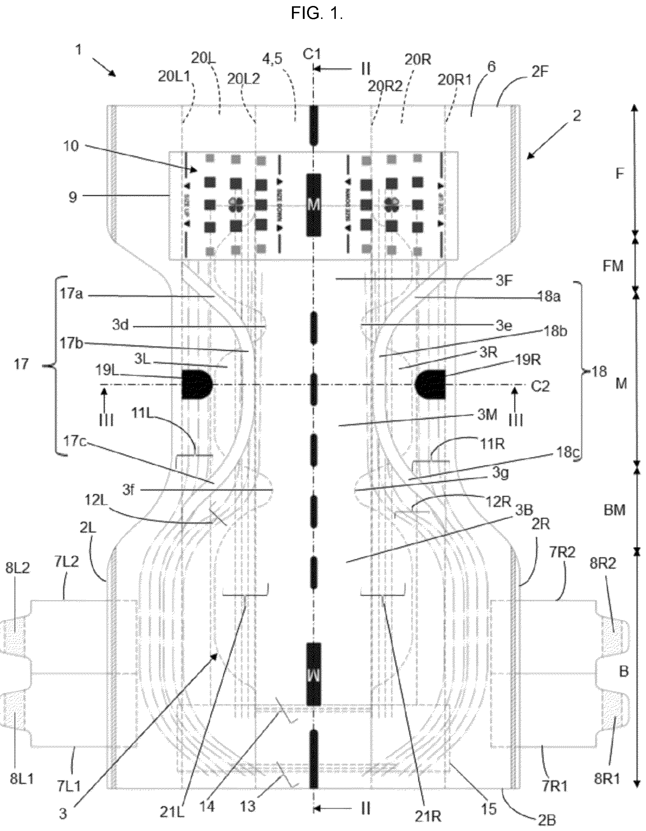

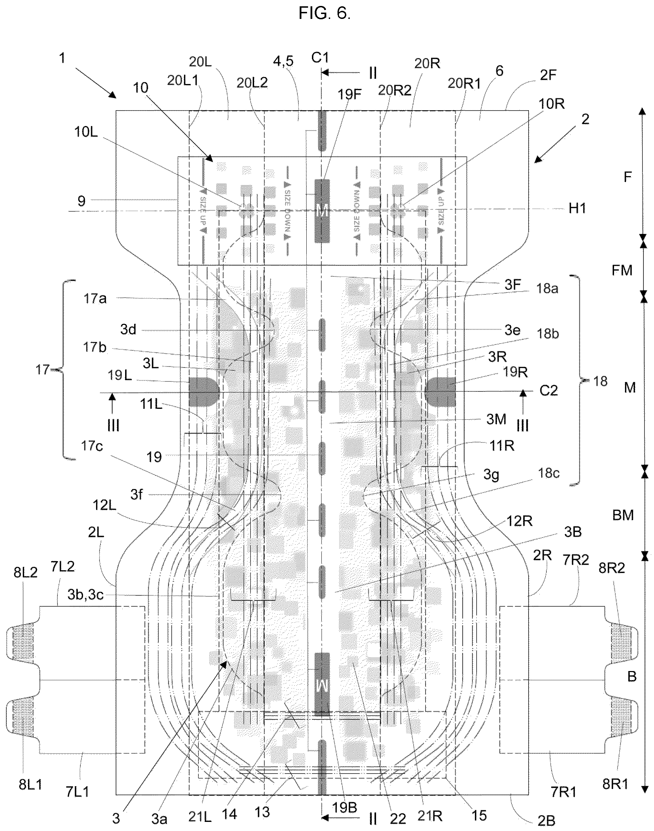

FIG. 1 is a bottom view of a bottom sheet of the disposable diaper of FIG. 6;

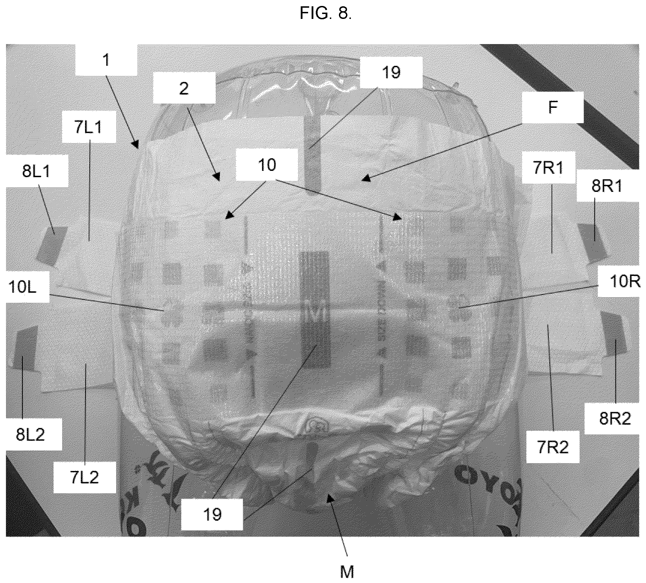

FIG. 8 is a photograph showing a front of a disposable diaper according to an embodiment of the present invention when the diaper is partially put on, with tapes deployed outward to the left and right; and

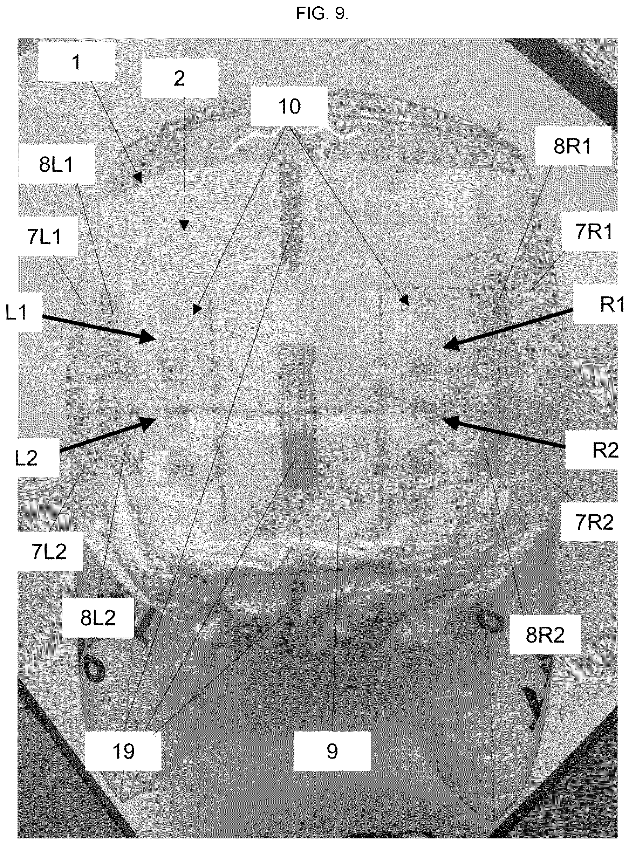

FIG. 9 is a photograph showing a front of a disposable diaper according to an embodiment of the present invention when the diaper is completely put on, with the tapes attached to an exterior surface of a body by fastening units.

### DESCRIPTION OF EMBODIMENTS

### <First embodiment>

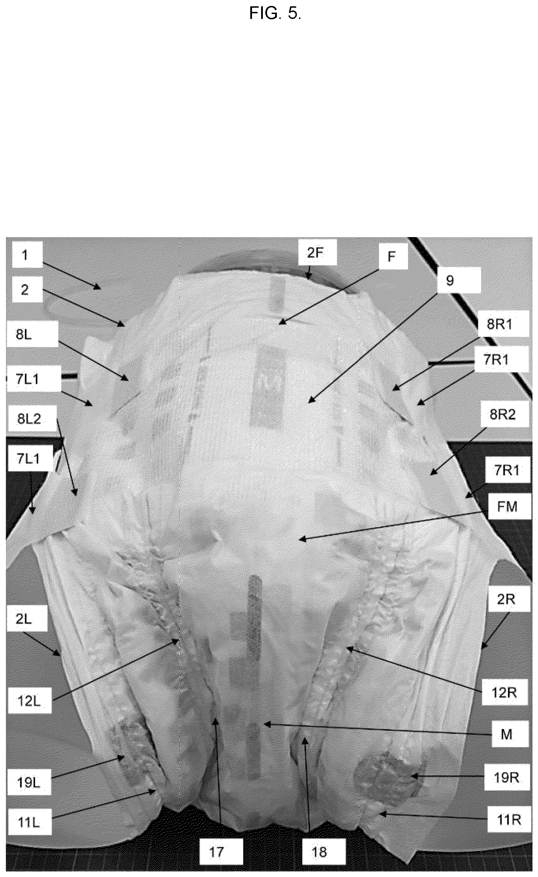

Referring to FIGs. 1 to 5, a disposable diaper according to a first embodiment of the present invention is described below.

As shown in FIG. 1, a disposable diaper 1 is a tape-type disposable diaper. A body 2 of the disposable diaper 1 has a longitudinal direction as a front-back direction and a width direction as a left-right direction. The body 2 has an overall shape in which a width of a middle section M of a longitudinal direction of a rectangle is narrowed. The body 2 has a front section F that is one end in the longitudinal direction and a back section B that is another end and is longer than the front section F. The body 2 has a front transition section FM that gradually narrows from the front section F to the middle section M and a back transition section BM that gradually narrows from the back section B to the middle section M.

The body 2 envelops a liquid-retentive absorber 3 with a plurality of sheets. The plurality of sheets includes a top sheet 4, a bottom sheet 5, and an exterior sheet 6.

The absorber 3 quickly absorbs liquid such as urine discharged from a wearer and holds it inside. The absorber 3 is made from raw materials including, for example, water-absorbent fiber obtained by defibrating a rolled sheet pulp and a powdery or granular superabsorbent polymer. These raw materials are air-conveyed to a molding die installed on the peripheral surface of the suction drum and are deposited and molded in the molding die while being sucked. The absorber 3 is made by sandwiching such a molded product 3a with water-absorbent papers 3b and 3c.

The top sheet 4 constitutes a wearer-facing surface of the body 2. The top sheet 4 is approximately rectangular in shape and covers the wearer-facing surface of the absorber 3, and both are secured with such as an adhesive. The top sheet 4 is permeable to liquid and allows liquids such as urine discharged from the wearer to permeate through it and move into the absorber 3.

The top sheet 4 is made of a liquid-permeable non-woven fabric, for example, a non-woven fabric made of chemical fibers such as polyethylene, polypropylene, polyester, nylon, and rayon, or natural fibers such as cotton, and cellulose, or a combination thereof. The top sheet 4 is not limited to the above-mentioned non-woven fabrics and may also be made of suitable liquid-permeable materials, such as a perforated film, a tissue, or a sheet of these materials joined together as appropriate.

The bottom sheet 5 is approximately rectangular in shape and covers the surface of the absorber 3 opposite the wearer-facing surface, and both are secured with such an adhesive. The bottom sheet 5 is impermeable to liquid and prevents excretions that have permeated the top sheet 4 or excretions that have not been absorbed or retained by the absorber 3 from leaking out.

The bottom sheet 5 is made of, for example, a liquid-impermeable film such as polyethylene, polypropylene, and polyester, or a liquid-impermeable nonwoven fabric. The bottom sheet 5 is not limited to the above-mentioned films or non-woven fabrics and may also be made of a suitable liquid-impermeable materials, such as an impermeable elastic material, a material coated with a waterproof adhesive, a laminate thereof, or a sheet of these materials joined together as appropriate.

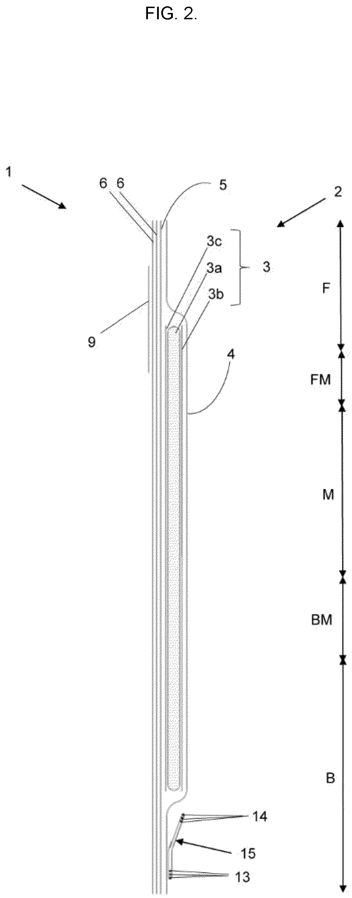

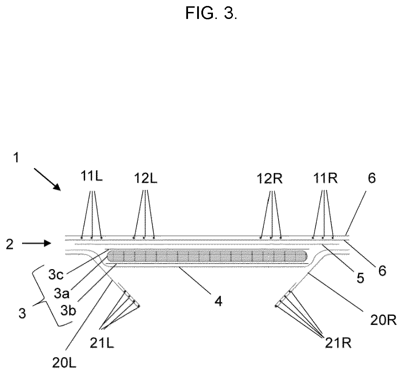

The exterior sheet 6 constitutes an exterior surface of the body 2. The exterior sheet 6 has the overall shape in which the width of the middle section M of the longitudinal direction of the rectangle is narrowed, just like the body 2. The exterior sheet 6 is arranged to further cover the bottom sheet 5 which covers the absorber 3, and the two are secured with such as an adhesive. As shown in FIGs. 2 and 3, in this embodiment, the two exterior sheets 6, 6 are arranged on top of the bottom sheet 5, and these three sheets are secured together with such as an adhesive.

The exterior sheet 6 is made of, for example, chemical fibers such as polyethylene, polypropylene, polyester, nylon, and rayon, or natural fibers such as cotton and cellulose, or a nonwoven fabric made of a combination thereof. The exterior sheet 6 may be less impermeable than the bottom sheet 5.

The disposable diaper 1 has two tapes 7 (7L1, 7L2, 7R1, 7R2) each on the left and right sides protruding outward from the left and right edges 2L and 2R of the back section B of the body 2. The tapes 7 (7L1, 7L2, 7R1, 7R2) are secured adjacent to the left and right edges 2L and 2R of the back section B of the body 2 by such an adhesive or heat welding.

The tape 7 is made of, for example, a non-woven fabric made of chemical fibers such as polyethylene, polypropylene, polyester, nylon, and rayon, or natural fibers such as cotton and cellulose, or a combination thereof. The tape 7 is not limited to the non-woven fabrics mentioned above. For example, the tape 7 may be made partly or entirely of an elastic material. When the tape 7 is made partly or entirely of an elastic material, the position at which the fastening unit 8 is attached to and detached from the exterior surface of the body 2 can be appropriately adjusted according to the elasticity of the tape 7 when the disposable diaper 1 is worn. This further improves the fit and size compatibility of the disposable diaper 1 to the wearer.

Each protruding end of the tape 7 (7L1, 7L2, 7R1, 7R2) is provided with a fastening unit 8 (8L1, 8L2, 8R1, 8R2) that is attachable to the exterior surface of the body 2 at the front section F or the front transition section FM. The fastening unit 8 that is attachable to the exterior surface of the body 2 can be selected according to the material of the exterior surface of the body 2. For example, if the material constituting the exterior surface of the body 2 is non-woven fabric, a hook-and-loop fastener with a group of hooks that can be attached to and detached from the material can be used. The fastening unit 8 may also be made of adhesive tape having adhesive properties.

A target tape 9 is provided on the surface of the exterior sheet 6 in the front section F and the front transitional section FM of the body 2 of the disposable diaper 1. The target tape 9 constitutes an exterior surface on which the fastening unit 8 (8L1, 8L2, 8R1, 8R2) can be attached and detached.

As shown in FIG. 1, the target tape 9, which constitutes the exterior surface of the front section F and the front transition section FM of the body 2, is printed with a target 10, which is a marker to assist in determining the position for attaching and detaching the fastening unit 8.

Furthermore, the disposable diaper 1 includes left and right outer elastic members 11L and 11R which are composed of a group of thread-like elastic materials arranged adjacent to the left and right edge 2L and 2R of the body 2, left and right inner elastic members 12L and 12R which are composed of a group of thread-like elastic materials arranged closer to a left-right centerline C1 between the left and right sides of the body 2 than the left and right outer elastic members 11L and 11, and a back end elastic member 13 arranged in the width direction adjacent to a back edge 2B of the back section B of the body 2. The back end elastic member 13 is arranged to couple between the left and right inner elastic members 12L and 12R.

As shown in FIG. 3, the left and right outer elastic members 11L and 11R and the left and right inner elastic members 12L and 12R are stretchably joined between the two exterior sheets 6 and 6 by such as an adhesive and heat welding. Without being limited to the above, for example, the left and right outer elastic members 11L and 11R or the left and right inner elastic members 12L and 12R may be joined between the exterior sheet 6 and the bottom sheet 5.

As shown in FIGs. 1 and 2, the disposable diaper 1 further includes a flap 15 whose base end is secured adjacent to the back edge 2B of the back section B of the body 2 and whose free end has a flap elastic member 14 arranged in the left-right direction. The back end elastic member 13 is provided within the base end of the flap15 and is joined with such as an adhesive so as to be stretchable in the left-right direction. The flap elastic member 14 is provided within the free end of the flap 15 and is joined with such as an adhesive so as to be stretchable in the left-right direction. The base end side of the flap 15 is secured to the back section B of the body 2, and the free end side of the flap 15 is capable of standing up.

The left and right outer elastic members 11L and 11R are arranged in the longitudinal direction along the left and right edges 2L and 2R in the middle section M of the body 2, are arranged obliquely along the left and right edges 2L and 2R in the back transition section BM, are arranged in the longitudinal direction along the left and right edges 2L and 2R in the back section B excluding the back end section, and are arranged obliquely toward the left-right centerline C1 in the back end section.

The left and right inner elastic members 12L and 12R are arranged in the longitudinal direction at a distance from the outer elastic members 11L and 11R in the middle section M of the body 2, are arranged obliquely toward the back section B so as to gradually approach the outer elastic members 11L and 11R in the back transition section BM, are arranged in the longitudinal direction along the outer elastic members 11L and 11R in the back section B excluding the back end section, and are arranged obliquely toward the left-right centerline C1 along the outer elastic members 11L and 11R in the back end section.

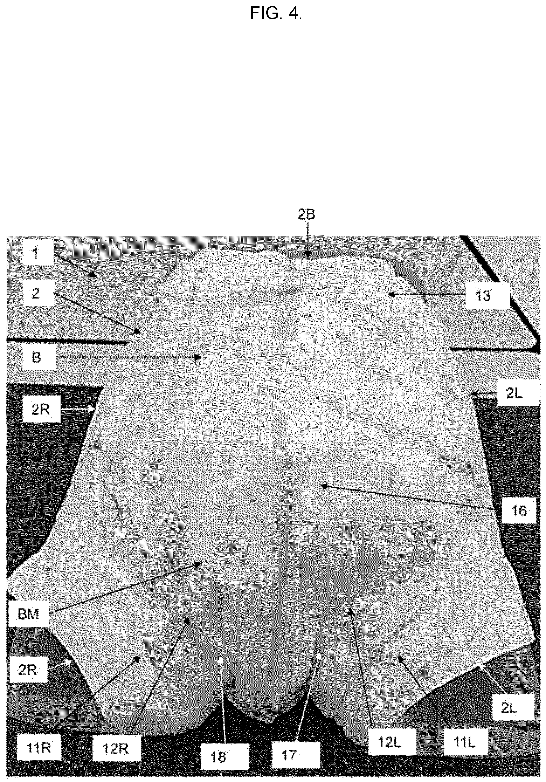

The back end elastic member 13 is arranged in the width direction so as to couple between the left and right inner elastic members 12L and 12R adjacent to the back edge 2B of the back section B of the body 2. As a result, the outer elastic members 11L and 11R, the inner elastic members 12L and 12R, and the back end elastic member 13 are arranged in an approximately arc shape at the back transition section BM and the back section B of the body 2. As shown in FIG. 4, the back transition section BM and the back section B of the body 2 fit along the wearer's buttocks when worn and include a bulging structure 16 that has an excrement storage space between them and the buttocks. As a result, the diaper can hold excrement even when a large amount of excrement is produced, more reliably preventing excrement leakage.

According to the disposable diaper 1, the portion where the outer elastic members 11L and 11R, the inner elastic members 12L and 12R, and the back end elastic member 13 are arranged in an approximately arc shape in the back transition section BM and the back section B of the body 2 fits along the rounded buttocks of the wearer, so that there is no gap between the diaper and the wearer ,and leakage of excrement can be prevented. Furthermore, a bulge structure 16 is provided inside the portion where the outer elastic members 11L and 11R, the inner elastic members 12L and 12R, and the back end elastic member 13 are arranged in the back transition section BM and back section B of the body 2, and the bulge structure 16 has an excrement storage space between its inner surface and the buttocks when worn, so that even if a large amount of excrement is produced, it can be held and leakage of excrement can be reliably prevented. In this way, it is possible to wear a tape-type disposable diaper like a pants-type diaper with a bulge structure.

If the disposable diaper 1 is not properly worn along the wear's groin, thigh bases and gluteal grooves, a gap is created between the diaper and the wearer, and the bulge structure 16 with a sufficient volume of the excrement storage space is not formed and maintained, resulting in the problem of leakage of the excrement held in the excrement storage. Therefore, it is important to properly wear the tape-type disposable diaper 1.

The disposable diaper 1 includes left and right guidelines 17 and 18 that are visible on the exterior surface of the front transition section FM, the middle section M and the back transition section BM of the body 2 and that are applied along the wear's groin, thigh bases and gluteal grooves. The left and right guidelines 17 and 18 have left and right straight portions 17b and 18b extending in the longitudinal direction at positions overlapping the left and right inner elastic members 12L and 12R in the middle section M as portions that are applied along the wearer's thigh bases. The left and right guidelines 17 and 18 have left and right back oblique portions 17c and 18c extending obliquely from positions overlapping the left and right outer elastic members 11L and 11R in the back transition section BM to the back ends of the left and right straight portions 17b and 18b as portions that are applied along the wearer's gluteal grooves. The left and right guidelines 17 and 18 have left and right front oblique portions 17a and 18a extending obliquely from positions overlapping the left and right outer elastic members 11L and 11R in the front transition section FM to the front edge of the left and right straight portions 17b and 18b as portions that are applied along the wearer's groins.

A method for putting the tape-type disposable diaper 1 on a wearer using the guidelines 17 and 18 is described below. First, the straight portions 17b and 18b of the left and right guidelines 17 and 18 are applied along the thigh bases, which are borders between the thigh and the groin of the wearer. Next, the left and right back oblique portions 17c and 18c are applied along the gluteal grooves, which are borders between the highs and buttocks of the wearer. Finally, the left and right front oblique portions 17a and 18a are applied along the groin of the wearer. After that, the fastening units 8 (8L1, 8L2, 8R1, 8R2) provided on the tapes 7 (7L1, 7L2, 7R1, 7R2) are attached and secured to the exterior surface of the front section F or the front transition section FM of the body 2.

According to the above-mentioned method, the disposable diaper 1 is put on the wearer while applying the guidelines 17 and 18 first to the wearer's thigh bases, then to the gluteal grooves, and finally to the groin. This makes it possible to easily and reliably realize that the portion, where the outer elastic members 11L and 11R, the inner elastic members 12L and 12R, and the back end elastic member 13 in the back transition section BM and the back section B are arranged, fits along the rounded buttocks of the wearer and that the bulge structure 16 with the excrement storage space between the inner surface of the portion and the buttocks is provided.

Furthermore, the disposable diaper 1 includes left and right center points 19L and 19R that are visible on the exterior surface of the body 2 and that indicate center positions of the straight portions 17B and 18B which are the portions of the left and right guidelines 17 and 18 and which are applied along the wearer's thigh bases.

In the above-mentioned method of putting the disposable diaper 1 on the wearer using the guidelines 17 and 18, when the straight portions 17b and 18b are applied along the thigh bases, which are the borders between the thighs and the crotch of the wearer, the left and right center points 19L and 19R allow the straight portions 17b and 18b to be easily and correctly identified.

As shown in FIGs. 1 and 3, the body 2 of the disposable diaper 1 includes left and right standing sheets 20L and 20R for standing at fold lines 20L1 and 20R1 along the left and right edges 2L and 2R in the middle section M. The left and right standing sheets 20L and 20R are secured to the top sheet 4 with such an adhesive between the fold line 20L1 and the left edge 2L, between the fold line 20R1 and the right edge 2R, along the front edge 2F, and along the back edge 2B. Unsecured portions of the left and right standing sheets 20L and 20R form standing units that standing from the top sheet 4 at the fold lines 20L1 and 20R.

Left and right standing elastic members 21L and 21R, which are composed of a group of thread-like elastic materials, are provided along free ends 20L2 and 20R2 of the left and right standing sheets 20L and 20R in the front-back direction. The left and right standing elastic members 21L and 21R are joined between two layers of the left and right standing sheets 20L and 20R folded back at the free ends 20L2 and 20R2 so as to be stretchable in the front-back direction.

When the left and right standing elastic members 21L and 21R contract in the front-back direction, the standing units of the left and right standing sheets 20L and 20R form gathers and stand up from the top sheet 4. Thus, when the disposable diaper 1 is worn, the standing units of the left and right standing sheets 20L and 20R are in close contact with the wearer so as to prevent excretions from leaking outward.

The left and right standing sheets 20L and 20R are made of, for example, a liquid-impermeable or water-repellent non-woven fabric, and may be made of the same raw material as the bottom sheet 5 or the exterior sheet 6.

The outer elastic members 11L and 11R, the inner elastic members 12L and 12R, the back end elastic member 13, the flap elastic member 14, and the standing elastic members 21L and 21R are made of, for example, stretchable materials such as natural rubber, polyurethane resin, and olefin-based elastomer.

As shown in FIG. 1, the absorber 3 has a front portion 3F, a middle portion 3M, and a back portion 3B that respectively correspond to the wearer's lower abdomen, crotch, and buttocks, and has left and right first recesses 3d and 3e with narrowed widths between the front portion 3F and middle portion 3M, and has left and right second recesses 3f and 3g with narrowed widths between the middle portion 3M and back portion 3B.

The middle portion 3M of the absorber 3 has left and right bulging portions 3L and 3R, which bulge outwardly between the left first and second recesses 3d and 3f, and between the right first and second recesses 3e and 3g. The left and right bulging portions 3L and 3R of the absorber 3 of the disposable diaper 1 are applied along the wearer's thighs so as to increase absorption capacity of the middle section M of the body 2 and to prevent leakage of excretions from the left and right of the middle section.

When the width between the roots of the left and right bulging portions 3L and 3R of the absorber 3 appropriately corresponds to the width of the wearer's crotch, the disposable diaper 1 fits the wearer's crotch better. The width between the roots of the left and right bulging potions 3L and 3R of the absorber 3 is the distance between a line connecting the innermost parts of the left first and second recesses 3d and 3f and a line connecting the innermost parts of the right first and second recesses 3e and 3g. The width between the two roots of the left and right bulging portions 3L and 3R of the absorber 3 is appropriately selected according to the size of the disposable diaper 1, and may be selected, for example, between 80 mm and 120 mm.

The group of thread-like elastic materials constituting the left and right inner elastic members 7L and 7R overlap the left and right bulging portions 3L and 3R of the absorber 3 and are arranged in parallel in the front-back direction. Thus, the left and right bulging portions 3L and 3R are supported by the left and right inner elastic members 7L and 7R, increasing the absorbing power of the middle portion 3M, thereby preventing lateral leakage of excrement.

### <Second embodiment>

Referring to FIGs. 6 to 9, a disposable diaper according to the second embodiment of the present invention is described below. Elements of the second embodiment using the same names and signs as those of the first embodiment is not described if they have the same as those of the first embodiment. FIG. 2 to 5 is also referenced if necessary for the description of the second embodiment.

As shown in FIG. 6, the disposable diaper 1 of the second embodiment is a tape-type disposable diaper. A centerline indicator 19 that is arranged in a dashed line on the left-right centerline C1 of the body 2, the left and right guidelines 17 and 18 that are applied along the wearer's groin, thigh bases and gluteal grooves, left and right reference marks 10L and 10R that are symmetrically arranged with respect to the left-right centerline C1 as a guide for an attachment position of the fastening units 8, a group of graphic marks 10 that are formed or regularly arranged symmetrically with respect to the left-right centerline C1 and a orthogonal line H1 to the left-right centerline C1 in the front section F of the body 2, the left and right center points 19L and 19R that indicate each center position of the left and right guidelines 17 and 18 in the longitudinal direction, and pattern 22 that expands to the middle section M, the back transition section BM and the back section B of the body 2 are visible on the exterior surface of the body 2 of the disposable diaper 1.

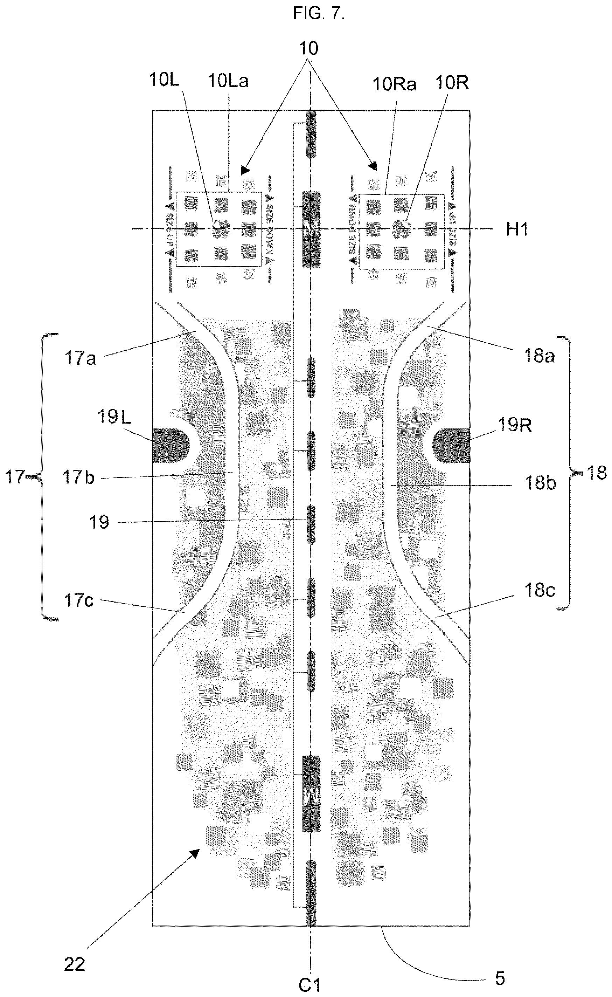

As shown in FIG. 7, the centerline indicator19, the left and right guidelines 17 and 18, the left and right reference marks 10L and 10R, a group of the graphic marks 10, the left and right center points 19L and 19R, and the pattern 22 are printed on the bottom sheet 5. As shown in FIG. 6, the centerline indicator 19 excluding a size-and-centerline indicator 19F described below, the left and right guidelines 17 and 18, the left and right center points 19L and 19R, and the pattern 22 are visible through the two exterior sheets 6 and 6 over the bottom sheet 5. The size-and-centerline indicator 19F, the left and right reference marks 10L and 10R, and a group of the graphic marks 10 are visible through the two exterior sheets 6 and 6 over the bottom sheet 5 and the target tape 9 secured on the sheets 6 and 6.

The group of graphic marks 10 serves as a guide for properly attaching the fastening units (8L1, 8L2, 8R1, 8R2) of the tapes 7 (7L1, 7L2, 7R1, 7R2) to the exterior surface of the front section F and the front transition section FM of the body 2. As shown in FIG.7, The group of the graphic marks 10 that is formed or regularly arranged symmetrically with respect to the left-right centerline C1 of the body 2 and the orthogonal line H1 to the left-right centerline C1. More specifically, the group of graphic marks 10 is composed of squares arranged in three rows on each side of the left-right centerline C1, with five squares per row. However, the marks 10L and 10R in the second row on the left and right on the orthogonal line H1 have a different shape from the squares, as described below. The further each of the graphic marks 10 is away from the left-right centerline C1, the larger the distance between the marks 10 from each other in the direction of the left-right centerline C1. Therefore, the marks 10 in the two left rows in front of the orthogonal line H1 are arranged obliquely from the front left to the back right, and the marks 10 in the two right rows in front of the orthogonal line H1 are arranged obliquely from the front right to the back left. The marks 10 in the two left rows behind the orthogonal line H1 are arranged obliquely from the back left to the front right, and the marks 10 in the two right rows behind the orthogonal line H1 are arranged obliquely from the back right to the front left.

The group of graphic marks 10 includes a pair of left and right reference marks 10L and 10R that are on the orthogonal line H1 and have features that distinguish them from other marks. The left and right reference marks 10L and 10R are in the shape of a four-leaf clover, and only the front left leaf of the left reference mark 10L and the front right leaf of the right reference mark 10R are blank. Thus, the left and right reference marks 10L and 10R have features that distinguish them from other marks, such as different shapes, sizes or colors, so that they stand out and serve as landmarks for determining the position for attaching the fastening unit 8 of the tape 7 to the exterior surface of the body 2. By using them as landmarks, it becomes easier to determine the appropriate attachment position for the wearer of the diaper 1 and to achieve the appropriate attachment position the next time and thereafter.

The left and right reference marks 10L and 10R, and the graphic marks 10La and 10Ra adjacent to the reference marks 10L and 10R are larger than the remaining graphic marks. Therefore, the reference marks 10L and 10R and their surrounding areas are easy to recognize, making it easy to pull up or down the tapes 7 relative to those areas so as to attach the fastening unit 8.

As shown in FIGs. 6 and 7, the centerline indicator 19 is disposed in a form of a dashed line on the left-right centerline C1 of the body 2. A size and centerline indicator 19F that intersects with the orthogonal line H1 in the front section F and a size and centerline indicator 19B that is approximately in the center of the back section B are components of the centerline indicator 19, are longer and wider than the other components, and are marked with the letter "M" indicating the diaper size. The centerline indicator 19 is visible on the exterior surface of the body 2, so that the position of the left-right centerline C1 of the body 2 can be easily recognized. Therefore, the centerline indicator 19 can be used as a reference for positioning the tape-type disposable diaper 1 when the diaper is attached to the human body.

The left and right guidelines 17 and 18 have left and right front oblique portions 17a and 18a, straight portions 17b and 18b, and back oblique portions 17c and 18c, which are portions that are respectively applied along the wearer's groin, thigh bases, and gluteal grooves. As shown in FIG. 6, the left and right guidelines 17 and 18 have the left and right straight portions 17b and 18b positioned to overlap with the left and right bulging portions 3L and 3R and the left and right inner elastic members 12L and 12R of the absorbent body 3, the left and right front oblique portions 17a and 18a positioned along the left and right side edges of the front portion 3F of the absorber 3, and the left and right back oblique portions 17c and 18c positioned along the left and right side edges of the back portion 3B of the absorber 3.

More specifically, in the left-right guidelines 17 and 18, the portions to be applied along the thigh bases of the wearer are the left and right straight portions 17b and 18b that extend in the longitudinal direction at the position overlapping the left and right inner elastic members 12L and 12R in the middle section M. The portions to be applied along the gluteal grooves of the wearer are the left and right back oblique portions 17c and 18c that extend obliquely from positions overlapping the left and right outer elastic members 11L and 11R in the back transition section BM to the back end of the left and right straight portions 17b and 18b. The portions to be applied along the groin of the wearer are the left and right front oblique portions 17a and 18a that extend obliquely from a position overlapping the left and right outer elastic members 11L and 11R in the front transition section FM to the front end of the left and right straight portions 17b and 18b.

As shown in FIGs. 6 and 7, the left and right center points 19L and 19R indicate the respective center positions in the longitudinal direction of the left and right guidelines 17 and 18, are approximately bullet-shaped, and are arranged with their arc-shaped portions facing each other. When the straight portions 17b and 18b of the left and right guidelines 17 and 18 are applied along the wearer's thigh bases, the left and right center points 19L and 19R allow the center positions of the straight portions 17b and 18b to be easily and accurately recognized.

The pattern 22 is printed on the bottom sheet 5 of the body 2, as shown in Figure 7, and expands over the middle section M, the back transition section BM and the back section B of the body 2, as shown in Figure 6, and is visible through the exterior sheets 6 and 6 constituting the exterior surface of the body 2. As shown in FIGs. 6 or 4, in the back transition section BM and back section B of the body 2, the pattern 22 is visible only on the exterior surface of the bulge structure 16 formed inside the left and right outer elastic members 11L and 11R, the left and right inner elastic members 12L and 12R, and the back end elastic member 13, which are provided around the wearer's buttocks. Therefore, when wearing the tape-type disposable diaper 1, the pattern 22 makes it easy to see an area where the bulge structure 16 is formed and makes it easy to put on the diaper body 2 at an appropriate position on the buttocks of the wearer.

Referring to FIGs. 8 and 9, when the disposable diaper 1 is put on a wearer, first the straight portions 17b and 18b of the left and right guidelines 17 and 18 are applied along the thigh bases, which are borders between the wearer's thighs and crotch. Next, the back oblique portions 17c and 18c of the left and right guidelines 17 and 18 are applied along the gluteal grooves, which are borders between the wearer's thighs and buttocks. Finally, the front oblique portions 17a and 18a of the left and right guidelines 17 and 18 are applied along the groin of the wearer. Then, the fastening units 8 (8L1, 8L2, 8R1, 8R2) of the tapes 7 (7L1, 7L2, 7R1, 7R2) is attached to the exterior surface of the front section F or the front transition section FM of the body 2. According to this wearing method, it is easy and reliable to allow the portion where the outer elastic members 11L and 11R, the inner elastic members 12L and 12R, and the back end elastic member 13 of the back transition section BM and the back section B of the body 2 are arranged to fit along the buttocks of the wearer, and to provide the bulge structure 16 having a sufficient volume of excrement storage space between the portion's inner surface and the buttocks.

As shown in FIG. 8, when the back section B of the body 2 of the disposable diaper 1 is placed under the buttocks of the human body, the middle part M is placed against the crotch, and the front section F is placed over the lower abdomen, the centerline indicator 19 is placed on the left-right centerline extending from the navel to the crotch of the wearer, it becomes easy to determine the appropriate position of the body 2. When the fastening unit 8 of the tape 7 is attached to the exterior surface of the body 2, as shown in FIG. 8, the left and right reference marks 10L and 10R are used as landmarks. As shown in FIG. 9, first, the fastening unit 8L2 is attached to the exterior surface while the lower left tape 7L2 is pulled up in an upper right direction L2, and the fastening unit 8R2 is attached to the exterior surface while the lower right tape 7R2 is pulled up in an upper left direction R2. Next, the fastening unit 8R1 is attached to the exterior surface while the upper left tape 7L1 is pulled down in a lower right direction 7L1, and the fastening unit 8R1 is attached to the exterior surface while the upper right tape 7R1 is pulled down in a lower left direction R1. This makes it easy to wear the body 2 in close contact with the human body and prevent excrement from leaking.

According to the disposable diaper 1 of the second embodiment, the centerline indicator 19 arranged on the left-right centerline C1 of the body 2, the left and right guidelines 17 and 18 that are applied along the wearer's groin, thigh bases, and gluteal grooves, and the left and right reference marks 10L and 10R that are arranged symmetrically with respect to the left-right centerline C1 as landmarks for the attachment position of the fastening units 8 are printed on the bottom sheet 5 of the body 2 and are visible through the exterior sheets 6 and 6 and the target tape 9 that constitute the exterior surface of the body 2. Thus, the centerline indicator 19 makes the position of the left-right centerline of the body 2 to be easily and accurately recognized and to be easily positioned on a left-right centerline of the wearer. Furthermore, the left-right guidelines 17 and 18 allow the body 2 to be easily applied along the wearer's groin, thigh bases, and gluteal grooves. Furthermore, the left and right reference marks 10L and 10R allow the appropriate attachment position of the diaper 1 on the wearer to be easily recognized and also to be easily reproduced the next time and thereafter. Therefore, it is possible to provide a tape-type disposable diaper that is easy to put on properly so as to prevent leakage of excrement.

According to the disposable diaper 1 of the second embodiment, the centerline indicator 19, the left and right guidelines 17 and 18, the left and right reference marks 10L and 10R, and the group of graphic marks 10 that are formed symmetrically or are regularly arranged symmetrically with respect to the left-right centerline C1 and the orthogonal line H1 of the left-right centerline C1 in the front section F and the front transition section FM of the body 2 are printed on the bottom sheet 5 of the body 2, and are visible through the exterior sheets 6 and 6 and the target tape 9 constituting the exterior surface of the body 2. Furthermore, the farther each of the group of graphic marks 10 is located from the left-right centerline C1, the greater the interval between them in the left-right centerline direction. Furthermore, the group of graphic marks 10 includes the left and right reference marks 10L and 10R that are larger and have a different shape than the other graphic marks 10. Furthermore, the left and right reference marks 10L and 10R allow the appropriate attachment position of the diaper 1 on the wearer to be easily recognized and also to be easily reproduced the next time and thereafter. Furthermore, first, the lower left tape 7L2 is pulled up in the upper right direction L2 while the fastening unit 8L2 is attached to the exterior surface, and the lower right tape 7R2 is pulled up in the upper left direction R2 while the fastening unit 8R2 is attached to the exterior surface. Next, the upper left tape 7L1 is pulled down in the lower right direction 7L1 while the fastening unit 8R1 is attached to the exterior surface, and the upper right tape 7R1 is pulled down in the lower left direction R1 while the fastening unit R1 is attached to the exterior surface. This makes it easy for the diaper body 2 to be worn in close contact with the human body and to prevent excrement from leaking.

According to the disposable diaper 1 of the second embodiment, the centerline indicator 19, the left and right guidelines 17 and 18, the left and right reference marks 10L and 10R, and the left and right center points 19L and 19R indicating the center positions in the longitudinal direction of the left and right guidelines 17 and 18, are printed on the bottom sheet 5 of the body 2, and are visible through the exterior sheets 6 and 6 constituting the exterior surface of the body 2. The left and right center points 19L and 19R allow the center positions of the left and right guidelines 17 and 18 to be easily and accurately recognized, so that the left and right guidelines 17 and 18 can be applied along the wearer's groin, thigh bases, and gluteal grooves.

According to the disposable diaper 1 of the second embodiment, the centerline indicator 19, the left and right guidelines 17 and 18, the left and right reference marks 10L and 10R, and the pattern 22 extending over the middle section M, the back transition section BM, and the back section B of the body 2 are printed on the bottom sheet 5 of the body 2, and are visible through the exterior sheets 6and 6 constituting the exterior surface of the body 2. Furthermore, in the back section B and the back transition section BM of the body 2, the pattern 22 is visible only on the exterior surface of the bulge structure 16 formed inside the elastic members 11L and 11R, 12L and 12R, and 13 that are provided to surround the wearer's buttocks. Therefore, when the disposable diaper 1 is worn, the pattern 22 makes it easy to visually recognize the area in which the bulge structure 16 is formed and makes it easy to wear the diaper body 2 at the appropriate position on the wearer's buttocks.

According to the disposable diaper 1 of the second embodiment, the body 2 includes the liquid-permeable top sheet 4, the liquid-impermeable bottom sheet 5, the absorber 3 enveloped between the sheets 4 and 5, and the exterior sheets 6 and 6 that are over the bottom sheet 5 and form the exterior surface of the body 2. The centerline indicator 19, guidelines 17 and 18, the marks 10, 10L and 10R, and the center points 19L and 19R are printed on the bottom sheet 5 and are visible through the exterior sheets 6 and 6. When the bottom sheet 5 to be printed and the exterior sheets 6 and 6 constituting the exterior surfaces are made of appropriate materials, a clear print can be obtained and is visible through the exterior surfaces.

According to the disposable diaper 1 of the second embodiment, the target tape 9, which is transparent or translucent and allows the fastening unit 8 to be attached and detached, is secured onto the exterior sheets 6 and 6 that constitute the exterior surface of the body 2. The centerline indicator, the guidelines, the marks, and the center points are visible through the target tape 9. In addition, the fastening unit 8 can be attached and detached regardless of the material of the exterior sheets 6 and 6 that constitute the exterior surface of the body 2.

### <Other embodiment>

The disposable diaper of the present invention is not limited to the above-described embodiment, and includes, for example, modified embodiments that can be understood from the above embodiment, and modified embodiments that do not change the essential characteristics of the present invention.

For example, the disposable diaper of the present invention is not limited to one provided with the target tape 9. The target tape 9 may be omitted, and the fastening unit 8 may be attached to and detached from the exterior sheet 6 that constitutes the exterior surface of the front section F and the front transition section FM of the body 2.

The target 10 is printed on the exterior sheet 6 that constitutes the exterior surface of the front section F and the front transition section FM of the body 2, and the target tape 9 may be omitted. A transparent target tape may be provided over the target 10 printed on the exterior sheet 6. The target 10 may be omitted.

The group of graphic marks 10 is not limited to squares, but may be circles, diamonds, or other shapes. Also, the group of graphic marks 10 is not limited to being provided in three rows on each side from the left-right centerline C1 of the body 2, but may be provided in four or five rows on each side.

The centerline indicator, the guidelines, the marks, the center points, and the pattern may be printed on the exterior sheets 6 constituting the exterior surface of the body 2.

The disposable diaper of the present invention may not include the exterior sheet 6, and the bottom sheet 5 may constitute the exterior surface of the body 2. In this case, the bottom sheet 5 is preferably made of, for example, a non-woven fabric that is liquid-impermeable and to which the fastening unit 8 can be attached and detached. The exterior surface of the bottom sheet 5 may be printed with the target 10 that assists in determining the attachment position of the fastening unit 8 or may have the target tape 9 with the target 10 printed thereon secured thereto.

The back end elastic member 13 may be disposed directly in the width direction adjacent to the back edge 2B of the back section B of the body 2, without the flap and the flap elastic member 14.

The left and right guidelines 17 and 18 may include at least the left and right straight portions 17b and 18b that extend in the longitudinal direction at the position overlapping the left and right inner elastic members 12L and 12R in the middle section M, which are the portions to be applied along the wearer's thigh bases, and the left and right back oblique portions 17c and 18c that extend obliquely from the position overlapping the left and right outer stretch members 11L and 11R in the back transition section BM to the back ends of the left and right straight portions 17b and 18b, which are portions that are applied along the wearer's gluteal grooves. This makes it easy to fit the portion, where the outer elastics members 11L and 11R, the inner elastics members 12L and 12R, and the back end elastic member 13 in the back transition section BM and the back section B of the body 2 are arranged, along the rounded buttocks of the wearer, and makes it easy to provide the bulge structure 16 having an excrement storage space between the inner surface of the portion and the buttocks.

The left and right guidelines 17 and 18 do not necessarily have the left and right upper oblique portions 17a and 18a that extend obliquely from the positions overlapping the left and right outer elastic members 11L and 11R in the front transition section FM to the front ends of the left and right straight portions 17b and 18b, which are the portions that are applied along the wearer's groin. However, if the left and right upper oblique portions 17a and 18a are further provided, this makes it easier and more certain to fit the portion, where the outer elastics members 11L and 11R, the inner elastics members 12L and 12R, and the back end elastic member 13 in the back transition section BM and the back section B of the body 2 are arranged, along the rounded buttocks of the wearer, and makes it easier and more certain to provide the bulge structure 16 having an excrement storage space between the inner surface of the portion and the buttocks.

The disposable diaper of the present invention does not have to include the left and right center points 19L and 19R. However, if the center points 19L and 19R are provided, when the straight portions 17b and 18b of the left and right guidelines 17 and 18 are applied along the thigh bases, which are boaders between the thighs and crotch of the wearer, during the process of putting on the disposable diaper, the left and right center points 19L and 19R allow the straight portions 17b and 18b to be easily and correctly recognized.

The disposable diaper of the present invention may not have the centerline indicator 19, the group of graphic marks 10, the reference marks 10L and 10R, or the pattern 22.

The disposable diaper of the present invention does not have to include the left and right standing sheets 20L and 20R and the standing elastic members 21L and 21R. The disposable diapers without them are appropriate, for example, when they are used for wearers with low urine volume, or when the structure of the diaper should be simplified and manufactured inexpensively.

In the disposable diaper of the present invention, the absorber 3 does not have to include the two left and right recesses 3d, 3e, 3f, and 3g and the left and right bulging portions 3L and 3R between the recesses. Also, the left and right inner elastic members 12L and 12R may not be disposed adjacent to the base of the left and right bulging portions 3L and 3R.

### INDUSTRIAL APPLICABILITY

The present invention can be used to provide a tape-type disposable diaper that has excellent fit when worn and has a sufficient volume of excrement storage space to prevent leakage of excrement.

The present invention can also be used to provide a tape-type disposable diaper that is easy to wear properly so as to prevent leakage of excrement.

### REFERENCE SIGNS LIST

1: disposable diaper
2: body
2F: front edge
2B: back edge
2L: left edge
2R: right edge
3: absorber
3d and 3f: left recesses
3e and 3g: right recesses
3L and 3R: bulging portion
4: top sheet
5: bottom sheet
6: exterior sheet
7 (7L1, 7R1, 7L2, 7R2): tape
8 (8L1, 8R1, 8L2, 8R2): fastening unit
9: target tape
10: target (a group of graphic marks)
10L and 10R: reference mark
11L and 11R: outer elastic member
12L and 12R: inner elastic member
13: back end elastic member
14: flap elastic member
15: flap
16: bulge structure
17 and 18: guideline
17a and 18a: front oblique portion
17b and 18b: straight portion
17c and 18c: back oblique portion
19: centerline indicator
19L and 19R: center point
20L and 20R: standing sheet
20L2 and 20R2: free end
21L and 21R: standing elastic member
22: pattern
F: front section
FM: front transition section
M: middle section
BM: back transition section
B: back section
C1: left-right centerline
H1: orthogonal line to the left-right centerline

## Claims

1. A disposable diaper comprising:
a body that envelops an absorber with a plurality of sheets, the body having an overall shape in which a width of a middle section of a longitudinal direction of a rectangle is narrowed, the body having a front section that is one end in the longitudinal direction, a back section that is another end and is longer than the front section, a front transition section that gradually narrows from the front section to the middle section, and a back transition section that gradually narrows from the back section to the middle section;
tapes protruding outward from left and right edges of the back section of the body;
fastening units provided at protruding ends of the tapes and attachable to an exterior surface of the front section or the front transition section of the body;
outer elastic members comprising of a group of left and right thread-like elastic materials, the outer elastic members being arranged in the longitudinal direction along the left and right edges in the middle section of the body, being arranged obliquely along the left and right edges in the back transition section, being arranged in the longitudinal direction along the left and right edges in the back section excluding a back end section, and being arranged obliquely toward a left-right centerline of the body in the back end section;
inner elastic members comprising of a group of left and right thread-like elastic materials provided closer to the left-right centerline than the left and right outer elastic members, the inner elastic members being arranged in the longitudinal direction at a distance from the outer elastic members in the middle section of the body, being arranged obliquely toward the back section so as to gradually approach the outer elastic members in the back transition section, being arranged in the longitudinal direction along the outer elastic members in the back section excluding the back end section, and being arranged obliquely toward the left-right centerline along the outer elastic members in the back end section;
back end elastic members that are arranged in a width direction so as to couple between the left and right inner elastic members adjacent to the back edge of the back section of the body; and
left and right guidelines that are visible on the exterior surface of the front transition section, the middle section and the back transition section of the body and are applied along wearer's groins, thigh bases and gluteal grooves,
wherein the left and right guidelines have left and right straight portions extending in the longitudinal direction at positions overlapping the left and right inner elastic members in the middle section as portions that are applied along the wearer's thigh bases,
wherein the left and right guidelines have left and right back oblique portions extending obliquely from positions overlapping the left and right outer elastic members in the back transition section to the back ends of the left and right straight portions as portions that are applied along the wearer's gluteal grooves, and
wherein the back transition section and the back section of the body have a portion in which the outer elastic members, the inner elastic members and the back end elastic members are arranged in an approximately arc shape, which fits along the wearer's buttocks when worn and which provides a bulge structure with an excrement storage space between an inner surface of the portion and the buttocks.

2. The disposable diaper according to claim 1,
wherein the left and right guidelines have left and right front oblique portions extending obliquely from positions overlapping the left and right outer elastic members in the front transition section to the front edges of the left and right straight portions as portions that are applied along the wearer's groins.

3. The disposable diaper according to claim 1,
wherein a center point indicating a center position of each of the portions that are applied along the wearer's thigh bases in the left and right guidelines is visible on the exterior surface of the body.

4. The disposable diaper according to claim 1,
wherein a centerline indicator located on the left-right centerline of the body is visible on the exterior surface of the body.

5. The disposable diaper according to claim 1,
wherein left and right reference marks located symmetrically with respect to the left-right centerline as landmarks for attachment positions of the fastening units are visible on the exterior surface of the body.

6. The disposable diaper according to claim 1,
wherein a group of graphic marks formed symmetrically or regularly arranged symmetrically with respect to the left-right centerline and an orthogonal line to the left-right centerline in the front section of the body are visible on the exterior surface of the body.

7. The disposable diaper according to claim 1,
wherein a pattern expanding from the middle section to the back section of the body is visible only on the exterior surface within the area surrounded by the left and right inner elastic members in the back section.

8. The disposable diaper according to claim 1,
further comprising a target tape where the fastening unit is attachable and is provided on the exterior surface in the front section or the front transition section of the body.

9. The disposable diaper according to claim 1,
further comprising a flap whose base end is secured adjacent to the back edge of the back section of the body and whose free end has a flap elastic member arranged in the left-right direction,
wherein the back end elastic member is provided within the base end of the flap.

10. The disposable diaper according to claim 1,
further comprising left and right standing sheets for standing on each line along the left and right edges of the middle sections of the body; and
left and right standing elastic members arranged along each free edge of the left and right standing sheets.

11. The disposable diaper according to claim 1,
wherein the absorber has two recesses and a bulging portion between the recesses on each of the left and right edges.

12. The disposable diaper according to claim 1,
wherein the absorber has two recesses and a bulging portion between the recesses on each of the left and right edges, and
the left and right inner elastic members are arranged adjacent to the base of the left and right bulging portions.
